Europäisches Patentamt

European Patent Office

Office européen des brevets

(11) Publication number: **0 330 797**
**A2**

# EUROPEAN PATENT APPLICATION

(21) Application number: **88500100.8**

(22) Date of filing: **27.10.88**

(51) Int. Cl.⁴: **A61N 1/06 , A61B 17/39**

The title of the invention has been amended (Guidelines for Examination in the EPO, A-III, 7.3).

(30) Priority: **04.03.88 ES 8800658 U**

(43) Date of publication of application:
**06.09.89 Bulletin 89/36**

(84) Designated Contracting States:
**AT BE DE GB NL SE**

(71) Applicant: **INDIBA, S.A.**
**Ronda del Guinardo 64**
**E-08025 Barcelona(ES)**

(72) Inventor: **Jodra Hernandez, Epifanio c/o Indiba, S.A.**
**Ronda del Guinardo 64**
**E-08025 Barcelona(ES)**
Inventor: **Calbet Benach, José c/o Indiba, S.A.**
**Ronda del Guinardo 64**
**E-08025 Barcelona(ES)**

(74) Representative: **Pastells Teixido, Manuel**
**c/o PASTELLS TEIXIDO, S.L. Pau Claris 138, 5-1a**
**E-08009 Barcelona(ES)**

(54) **A device for cellular regeneration with high frequency currents.**

(57) Appliances for these purposes are known having an active electrode connected to a high frequency electronic circuit, the object of this invention being to avoid the overheating thereof and the troublesome electric discharges to the patient, mainly in case of breakdown of the insulating layer covering the active electrode, which transmits current capacitively. To this end, the active surface of the electrode (1) is protected by a thin resistant conductive layer (5); on the general circuit output transformer (11), operating at a frequency of 0.8 to 1 megacycle per second, there is disposed a metal section (12) as heat sink facing a fan (13), and a capacitor (14) is inserted in the terminal shunt (M) of said transformer (11) secondary winding, before connection thereof to the tool (4) carrying the active electrode (1).

Fig. 1

## Electronic Apparatus for medical and cosmetic therapy

The invention relates to an electronic apparatus for medical and cosmetic therapy.

The applicant has been experimenting on and manufacturing electronic devices for depilation and skin regeneration since 1984. These devices use pincers or an active electrode connected to a high frequency electronic circuit and comprise a return or neutral electrode applicable to the epidermis. These devices operate perfectly, and the object of the present invention is to improve them so as to improve and extend their application to patients in medicine in general, particularly in the treatment of pain and in dermatology. In the cosmetics field, the main purpose is for the regeneration of the skin and also to obtain a longer operative life of the apparatus without overheating. A further object is to avoid the troublesome electric discharges which could be given by the active electrode to the patient should by any chance the insulating layer covering said active electrode which transmits capacitive current become broken down. Such breakdown may be caused by the corrosion affecting the electrode in certain cases when certain moisturizing creams which are specific for particular treatments are used for soft massage on the skin area to be treated.

To achieve these improvements, the output transformer of the general apparatus circuit is coupled to a heat sink formed by an angle bar or bar of any other appropriate section not varying the specific features of the coil. The heat sink is placed opposite a fan and a capacitor is inserted in the terminal shunt of the transformer secondary winding, before connection to the tool carrying the active electrode.

The insulated active surface of the active electrode is protected by a thin layer of resistant conductive material such as a metal.

The operating frequency of this apparatus is from 0.8 to 1 megacycle per second to achieve a lower dispersion while maintaining correct conductivity.

So as to facilitate an understanding of the invention, there is given a detailed description illustrated with drawings of one exemplary embodiment which does not limit the scope of the invention.

In the drawings:

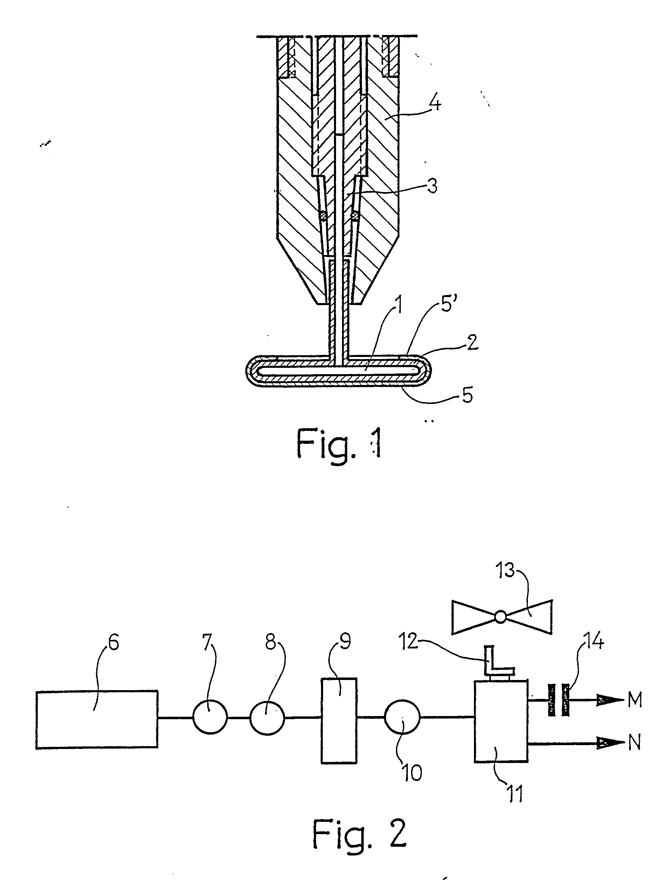

Figure 1 is an elevation view of the protected active electrode, attached to the handle forming the tool; and

Figure 2 is a schematical view of the main part of the general electronic circuit of the apparatus of the invention.

In the drawings, reference 1 is the active metal electrode which is externally electrically insulated by a layer of appropriate plastics material 2 and which is removably attached to a chuck member 3 mounted axially in the handle 4 forming the tool. This layer of plastics material, as desired, may be protected with a thin layer 5 of conductive material, i.e. metal, having an approximate thickness of 0.1 to 0.3 mm, and which is attached to the electrode by any appropriate system. For example, said thin metal layer may form a swaged capsule which will be clamped by the edges 5′ thereof on the rear face of the electrode. This metal layer may also be attached by any appropriate adhesive or be produced by electrolysis.

The general circuit comprises mainly the oscillator stage 6, the output of which is connected to a pre-amplifier stage 7 providing the necessary reinforcement for the signals sent to the driver transistor 8 which delivers them to the resonant circuit 9 forming a radiofrequency transformer for coupling to the power amplifier transistor 10 forming the output stage completed by the high frequency transformer 11, to which there is attached a heat sink formed by a metal angle bar 12, for example of aluminium, brass or copper, which is placed facing a fan 13.

The secondary winding of the transformer has one of its terminal shunts M connected through a capacitor 14 to the handle 4 carrying the active electrode, and the other one N to the neutral electrode in the apparatus. The anti-stimulus capacitor 14 will prevent the patient from feeling the sensation of direct passage of the electric current, should the active face of the active electrode be broken down.

This apparatus is completed with a timer to indicate the end of the session time in each treatment, which timer may be connected to the general circuit of the apparatus to switch off the active electrode.

Since the inclusion of the thin metal layer 5 to the active electrode slightly reduces the capacitive effect, so as not to lose efficiency in the treatment, the power shall be increased slightly and thus good results will also be obtained.

## Claims

1.- An electronic apparatus for medical and cosmetic therapy, of the type comprising a tool carrying an electrode connected to a high frequency electronic circuit, characterized in that the active surface of the electrode (1) transmitting current capacitively is electrically insulated (2) and protected against attack and corrosion by way of a thin layer (5) of resistant conductive material such as metal avoiding fortuitous breakdown of the insulation and direct passage of the current to the patient's skin, and in that the general circuit output stage transformer (11) comprises as heat sink, to reduce the inductance with a view to increasing the resonant frequency, a metal angle bar (12) or bar of other appropriate section facing by a fan (13).

2.- An electronic apparatus for medical and cosmetic therapy, according to claim 1, characterized in that a capacitor (14) is inserted in the terminal shunt (M) of the output stage transformer (11) secondary winding before the connection thereof to the tool (4) carrying the active electrode (1), to protect the patient from electrical discharge in case of breakdown of the insulation (2) of the active surface of said electrode, the general circuit operating at a frequency of 0.8 to 1 megacycle per second.

EP 0 330 797 A2

Fig. 1

Fig. 2